(19)

**Europäisches Patentamt**
**European Patent Office**
**Office européen des brevets**

(11) **EP 4 244 647 B1**

(12) **EUROPEAN PATENT SPECIFICATION**

(45) Date of publication and mention
of the grant of the patent:
**25.12.2024  Bulletin 2024/52**

(21) Application number: **21811012.0**

(22) Date of filing: **12.11.2021**

(51) International Patent Classification (IPC):
**G01S 7/292** *(2006.01)*     **A61B 5/024** *(2006.01)*
**A61B 5/0507** *(2021.01)*     **A61B 5/08** *(2006.01)*
**A61B 5/00** *(2006.01)*     **G01S 7/41** *(2006.01)*
**G01S 13/10** *(2006.01)*     **G01S 13/42** *(2006.01)*
**G01S 13/58** *(2006.01)*     **G01S 13/72** *(2006.01)*
**A61B 5/11** *(2006.01)*     **G01S 13/02** *(2006.01)*

(52) Cooperative Patent Classification (CPC):
**G01S 13/10; A61B 5/024; A61B 5/0507;**
**A61B 5/0816; A61B 5/1113; A61B 5/7264;**
**G01S 7/2923; G01S 7/415; G01S 7/417;**
**G01S 13/42; G01S 13/582; G01S 13/726;**
G01S 2013/0245

(86) International application number:
**PCT/EP2021/081483**

(87) International publication number:
**WO 2022/101390 (19.05.2022 Gazette 2022/20)**

(54) **OPTIMIZATION OF DATASET**

DATENSATZOPTIMIERUNG

OPTIMISATION D'ENSEMBLE DE DONNÉES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **13.11.2020  GB 202017922**

(43) Date of publication of application:
**20.09.2023  Bulletin 2023/38**

(73) Proprietor: **Elotec AS**
**7340 Oppdal (NO)**

(72) Inventor: **KLEVEN, Jan**
**7340 Oppdal (NO)**

(74) Representative: **Bryn Aarflot AS**
**Patent**
**Stortingsgata 8**
**0161 Oslo (NO)**

(56) References cited:
**CN-A- 110 058 220     CN-A- 111 142 102**
**US-A1- 2018 279 884     US-A1- 2019 094 350**

**US-A1- 2020 237 252**

• **TSAI YU-CHIAO ET AL: "High Accuracy**
**Respiration and Heart Rate Detection Based on**
**Artificial Neural Network Regression", 2020 42ND**
**ANNUAL INTERNATIONAL CONFERENCE OF**
**THE IEEE ENGINEERING IN MEDICINE &**
**BIOLOGY SOCIETY (EMBC), IEEE, 20 July 2020**
**(2020-07-20), pages 232 - 235, XP033815264, DOI:**
**10.1109/EMBC44109.2020.9175161**
• **PEGORARO JACOPO ET AL: "Multiperson**
**Continuous Tracking and Identification From**
**mm-Wave Micro-Doppler Signatures", IEEE**
**TRANSACTIONS ON GEOSCIENCE AND**
**REMOTE SENSING, IEEE, USA, vol. 59, no. 4, 10**
**September 2020 (2020-09-10), pages 2994 - 3009,**
**XP011845240, ISSN: 0196-2892, [retrieved on**
**20210324], DOI: 10.1109/TGRS.2020.3019915**

**(Cont. next page)**

- **ZHAO PEIJUN ET AL: "mID: Tracking and Identifying People with Millimeter Wave Radar", 2019 15TH INTERNATIONAL CONFERENCE ON DISTRIBUTED COMPUTING IN SENSOR SYSTEMS (DCOSS), IEEE, 29 May 2019 (2019-05-29), pages 33 - 40, XP033599299, DOI: 10.1109/DCOSS.2019.00028**

## Description

### Technical Field

The present invention relates to a method of optimising a dataset in order to detect body micro movements of one or multiple subjects due to respiration and heartbeat at a long distance, in a noisy environment and with improved filtering against interferences.

The invention exploits a technology based on radar in the mm wave band with a frequency range of 60 - 81 GHz. phased array-technology.

The invention is based on the creation of a 3D cloud-data output from a radar scan of the environment where one or more patients are situated.

### Background Art

KR102091974B1 describes a method and apparatus for tracking respiration and heart rate using an FMCW radar arranged at a distance from persons within a space. The radar may operate on a frequency of 60 or 77 GHz. A fast Fourier transform is applied on the received data and a tracking method comprising selecting a target range bin is applied to detect the location of persons. The chest movements due to respiration and heartbeat of the detected persons are measured by phase changes and distance fluctuations. A breathing pattern or a heart rate pattern may be determined through a frequency spectrum over time in a range bin. An abnormal breathing pattern is determined by expanding the signal strength.

US8870785B2 describes a device and method for contactless respiration monitoring of a patient. It is based on the Doppler-Radar principle and operates at frequencies below 30 GHz. The received signal is phase shifted and vectors are determined at different point in time. These vectors are defined by the time derivatives of the received signal and the phase shifted signal. The vectors provide an indicator of the change of respiration of the patient.

KR101895324B1 describes a method for measuring respiration rate and heartbeat using an ultra-wideband impulse radar signal. The received signal is run through band-pass filters corresponding to heartbeat and respiration frequency bands. CZT (Chirp Z-transform) is applied to the band-pass filtered signals to convert the signals into the frequency axis band.

CN209863802U describes a radar-based non-contact vital sign measurement system using the echo from a radar signal. A Fourier transform is done on the received signal, but how the vital signs are detected from this is poorly described.

KR102061589B1 describes a non-contact type living body monitoring and care system using ultra-wide band in a frequency band of 2-10 GHz. The received signal is run through a noise filter after which the distance of movement of the target is measured. From this respiratory state is analysed using 2D of 3D shaping.

US2008294019 describes a monitoring system using WLAN receivers operating as Doppler radar to detect heartbeat or respiration motion of a chest of a patient. An analyser is coupled to one of the transmitter and receiver to determine heart attack or stroke attack. US2018078216 describes a method for estimating a disease state for a patient using a non-invasive medical device that communicates with a network. US2019209022 describes a wearable electronic device, which includes a hybrid wireless communication module to acquire location data from both indoor and outdoor sources, as well as to selectively transmit an LPWAN signal to provide location information based on the acquired data.

GB2449081 describes a device for remote monitoring of breathing movements of a patient using a microwave radar. The radar is working on a frequency of 25 GHz. A processor mixes the received signal with the transmitted signal and with a signal in quadrature with the transmitted signal to determine a breathing frequency of the patient. In some embodiments, two signals at different frequencies are emitted toward the patient and processed in order to compensate for breathing patterns that are not uniquely resolvable at a single frequency.

WO2017002103 describes a system using a radio frequency interferometer antenna array where the received signals are run through a clutter cancellation module to filter out static non-human related echo signals. From the filtered signals are extracted a quantified representation of position postures, movements, motions and breathing of at least one human located within the specified area. In one embodiment the human position and posture is found by dividing the surveillance space into voxels (small cubes) in cross range, down range and height. Then is the reflected electromagnetic signal from a specific voxel estimated by a back projection algorithm, and the human position is estimated by averaging the coordinates of the human reflecting voxels for each baseline, triangulating all baselines' position to generate the human position in the environment. The human posture is estimated by mapping the human related high-power voxels into the form-factor vector, and finally tracking the human movements in the surveillance space.

US20150369911 describes a system for sensing human breathing at a distance. It is using a radar antenna. A clutter suppression is performed on the received data, where after one or more targets are detected in specific range bins. A breathing signal, including breathing rate, for the detected one or more targets is extracted. It is verified that each of the one or more targets are in associated range bins based on the extracted parameters. To estimate the target's range bin and distinguish between multiple targets, a combination of two signal processing techniques may be used (Singular Value Decomposition (SVD) and skewness).

EP3641649 describes a system and method for breathing monitoring using a Doppler radar-based sensor system. The frequency of the radar is in the range between 15 GHz and 130GHz, but preferably a frequency of about 60 GHz is used. The received signal is autocorrelated and run through a Fourier transform. The value of at least two peaks of the calculated Fourier transform are determined, and from this a breathing characteristic of the person is determined.

Some of the above systems and devices are at least partially depending on the patient being connected to or wearing a device, which inevitably will limit the freedom of movement of the patient and/or the time over which the patient can be monitored.

The radar that is proposed used in the system of the invention has a built-in method for estimation of heart rate (HR) and respiration rate (RR) on an integrated circuit built by Texas Instruments. However, it has been found that this circuit stops providing estimates on both HR and RR when the distance from the radar to the subject exceeds 1 meter. Consequently, the built-in abilities are too poor to give the radar any usefulness in monitoring patients unless they are very limited in their freedom of movement.

US 20200300972A1, filed by Origin Wireless, achieves good results on RR and HR detection and estimation for short distances (up to 2m). However, the reference does not mention detection and estimation on longer distances than this. The reference also mentions several challenges concerning multi-person respiration detection with 60 GHz.

Prior art methods used to detect and estimate respiration are in general based on traditional signal processing. These methods have certain limitations:

- Limited ability to detect respiration and/or heartbeat from longer distances
- The focal area for detection needs to be precisely configured
- The estimated respiration and/or heartbeat rate may deviate significantly from actual respiration rate under difficult conditions
- The respiration and/or heartbeat rate is vulnerable to noise from sources such as other persons or objects in motion. Most of the prior art systems referred to above are not able to simultaneously follow multiple subjects within the detection area.

CN 111142102 describes a system for detecting respiration of a patient using a MIMO radar. A clustering technique is used to determine which data points belong to the same person. Subsequently the amplitude of the points is determined by weighting the points that lies around the peak amplitude.

US 2020/237252 describes a patient monitoring device. US 2019/094350 describes a method for presence detection. US 2018/279884 describes a system for detecting respiration and heartbeat of a patient by using a mm radar.

In the article 'Y. -C. Tsai et al.: "High Accuracy Respiration and Heart Rate Detection Based on Artificial Neural Network Regression", 2020 42nd Annual International Conference of the IEEE Engineering in Medicine & Biology Society (EMBC), pages 232-235, DOI:10.1109/EMBC44109.2020.9175161', is described a system for detecting respiration and heartbeat of a patient by using a radar operating at a frequency of 24 GHz. The extraction of heart rate or respiration rate is done by various types of machine learning. The articles 'J. Pegoraro et al, "Multiperson Continuous Tracking and Identification From mm-Wave Micro-Doppler Signatures", IEEE Transactions on Geoscience and Remote Sensing, vol. 59, no. 4, pages 2994-3009, ISSN: 0196-2892, DOI:10.1109/TGRS.2020.3019915' and 'P. Zhao et al., "mID: Tracking and Identifying People with Millimeter Wave Radar", 2019 15th International Conference on Distributed Computing in Sensor Systems (DCOSS), pages 33-40, DOI: 10.1109/ DCOSS.2019.00028' disclose people tracking.

All of the above prior art references will process all of the data points that are determined to belong to the same person in the same way. This requires a certain processing capacity. Inevitably, the number of data points that can be processed is limited by the available processing capacity. This may influence on the accuracy of the respiration or heartbeat monitoring or the possibility to follow the person when this is moving. It may also not be possible to monitor several persons at once, and if possible, the accuracy of the measurements will be lower.

**Summary of invention**

An objective of the invention is to optimize a dataset from a phased-array radar to detect movement and orientation of subjects within a coverage area of the radar. This is achieved by the features of subsequent claim 1.

In a preferred embodiment, the method of the invention achieves the above objectives by:

1. The availability of a 3D point cloud data of high resolution, containing Doppler information about motions in different points.

2. Dynamic noise filtering.

3. The use of DBSCAN to separate point data into relevant groups.

4. Aggregating the grouped data into a single summary signal value

5. Extracting respiration rate from the group signal by applying signal processing techniques such as detrending, Butterworth filtering and multitaper method.

6. Combining frequency extraction with state-of-the art machine learning modelling methods through ensemble learning.

7. Efficiency of detecting respiration at significant distances (at least up to 5.5 meters)

8. Efficiency of detecting respiration in scenarios with added noise.

9. Demonstrated successful detection of respiration signal for multiple persons simultaneously.

A key item in the application of the method of the invention is a radar that is capable of detecting the presence and movement of subjects within a coverage space of the radar.

**Brief description of drawings**

Figure 1 shows a block diagram illustrating a flow chart of the respiration detection optimization algorithm of the present invention,

Figure 2 shows an illustration of the principle of phased-array antennas,

Figure 3 illustrates a simple example of a 3D point cloud based on raw data,

Figure 4 illustrates a number of data points and their association with point groups,

Figure 5 illustrates of how gradient boosting works with decision trees.

Figure 6 illustrates the framewise aggregation of grouped points to a time signal,

Figure 7illustrates focusing of the radar by applying different delays to each element of a phased array,

Figures 8a, 8b and 8c illustrate dynamic focusing of the radar across three consecutive frames,

Figure 9 shows an example of a 3rd order Butterworth filter that may be used for filtering the dataset,

Figure 10 shows a signal before filtering with the filter in figure 9,

Figure 11 shows a signal after being filtered with the filter in figure 9,

Figure 12 shows a multitaper spectral density estimate derived from a test subject with a respiration rate of 13 breaths per minute,

Figure 13 shows a set-up for data collection in a machine learning procedure,

Figure 14 illustrates an ensemble learning method according to the invention,

Figure 15 shows an experimental set-up for testing of the methods of the invention,

Figure 16 shows a set-up for the experiments 1-5,

Figure 17 shows a set-up for experiment 6,

Figure 18 shows a set-up for experiments 7,

Figure 19 shows a set-up for experiment 8,

Figure 20 shows a table of the results from experiments 1-8,

Figure 21 shows a graph of difference in breathing rate at various distances,

Figure 22 shows differences in breathing rate with added noise in three of the experiments, and

Figure 23 illustrates a successful detection of two persons and respiration estimates of each person.

**Detailed description of the invention**

The following description will explain the technical details of the method of the invention.

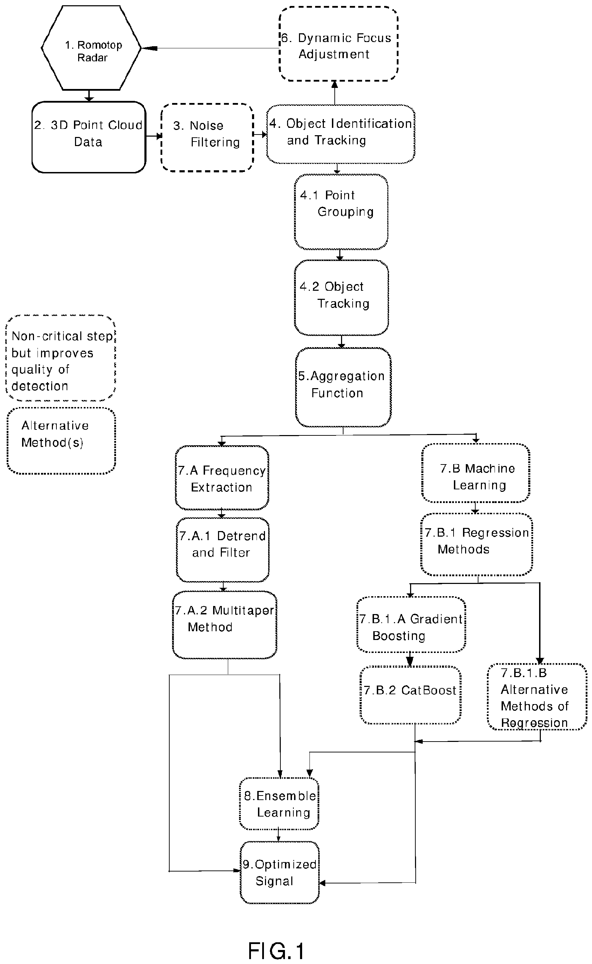

Figure 1 shows a block diagram illustrating a flow chart of the respiration detection optimization algorithm of the present invention.

The flow chart illustrates a method according to a preferred embodiment of the invention to determine a subject's respiratory rate with point clouds produced from radar sensors. The preferred method comprises nine steps: (1) Initialization and data acquisition from the radar configuration, (2) gathering of point cloud data with Doppler information, (3) noise filtering, (4) object identification and tracking of objects, (5) an aggregation function to group and form the grouped velocity data into a single summary signal, (6) dynamic focus of the radar based on object identification, (7) extraction of respiration signal by use of (7.A) traditional signal processing methods, (7.B) machine learning, and subsequently combining the two methods in step (7) with (8) ensemble learning, in order to obtain the (9) final optimized respiration signal. The different steps in the flow chart will be described in detail below.

The preferred radar to be used with the method of the present invention is a mm waveband radar, which operates at around 60GHz frequency. Waves with a frequency around this value will not penetrate walls, floors and ceilings between rooms to any substantial degree, and hence not be susceptible to detect persons in neighbouring rooms. However, the waves will penetrate clothes and blankets and thinner plates, such as glass and drywall board inside the room. This means that chest movements due to respiration and heartbeat of subjects, such as persons present within the coverage area of the radar, such as a room., will be detected.

The radar can conveniently be mounted in the ceiling of a room, such as a patient room, livestock room. This will in many scenarios provide good coverage of the room. The radar may also be mounted otherwise, such as on a wall, on or in the floor, on a separate mount. It may also be mounted in other areas where it is desired to detect respiration or heartbeat induced movements, such as in onshore, offshore and under water installations, in an automobile, bus, boat, submarine, aeroplane, train, tram or other type of vehicle.

It can also be used in an outdoor area.

The monitoring device is preferably capable of wireless communication, both with various sensors and with a computer system in a cloud or on the premises. Adequate encryption of data ensures safety from hacking. The wireless communication can conveniently be by the Bluetooth protocol. This has a relatively short range and hence limited interference with other wireless communications.

The radar employs the several antennas arranged in a phased-array manner, which enables the beam of the antennas to be controlled in different directions without moving the radar.

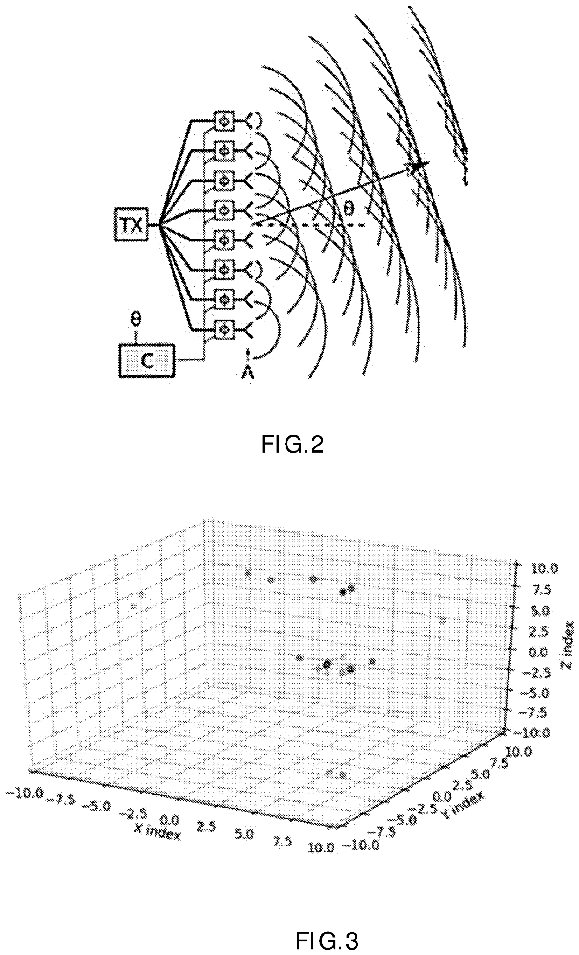

Figure 2 is an illustration of the principle of phased-array antennas. A row of antenna elements A is powered by a transmitter TX. The feed current for each antenna passes through a phase shifter ($\varphi$) controlled by a computer (C). The lines show the wave fronts of the radio waves emitted by each element. The individual wave fronts are spherical, but they superpose in front of the antenna to create a planar wave, i.e., a beam of radio waves travelling in a specific direction. The phase shifters delay the radio waves progressively going up the row of antennas, so each antenna emits its wave front a little later than the one below it. This causes the resulting planar wave to be directed at an angle $\theta$ to the antenna's axis. By changing the phase shifts $\Phi$ the computer can instantly change the angle $\theta$ of the beam. Figure 2 illustrates a one-dimensional array of antennas, but most phased arrays have twodimensional arrays of antennas instead of the linear array shown here, and the beam can be steered in two dimensions. The present invention will utilize a twodimensional array.

The phased-array technology makes it possible for the radar to output 3D point cloud-data with Doppler information about motions in different points within the 3D space covered. This is information that has not been possible to obtain by radars with a single antenna. The radar is illustrated as item 1 in figure 1.

The range resolution is heavily depending on the bandwidth. It has been found that a frequency of about 60 GHz supports a wider bandwidth range than radars that operate in smaller bandwidth areas, such as 4 GHz (UWB). The frequency can be up to about 81 GHz but should preferably be within the range of 60-64 GHz. This enables the radar according to the invention to detect objects and motions up to 20 times more accurately than UWB radars. The radar according to the invention facilitates accurate sensing with real-time decision making and complex signal processing, as will be explained below. Moreover, mm wave-based radars offer unique advantages because the sensors can detect movements through a variety of materials such as glass and drywall, which likely will be encountered in the intended application environments.

The phased-array radar is capable of creating a rich point-cloud data. One of the objects of the invention is to enable identification and separation of human motions from animals and mechanical objects and therefore reducing false detections to a great extent. Gathering point-cloud data with x, y and z co-ordinates with radial velocity provides a fine-grained range resolution. This level of resolution enables the radar to reliably detect and measure respiration movements from separate humans even though they are close to each other, like a doctor and a patient.

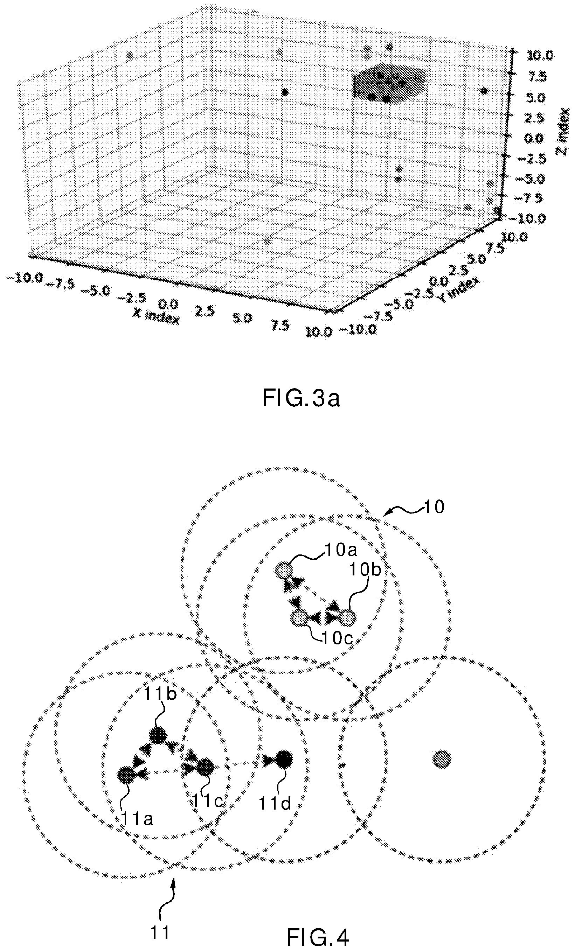

Figure 3 illustrates a simple example of a 3D point cloud based on raw data. Motion amplitude is denoted by grey level, i.e., the darker the point is, the higher the velocity is. The raw input signal consists of a sequence of 3D point cloud frames received at a rate of 20 frames/s from the radar.

Position and doppler-information, which includes velocity and direction of motion, of reflected points are obtained continuously by the sensors of the radar and is segmented into frames. Each frame is associated with a timestamp and contains a collection of point data. Item 2 in figure 1 illustrates the 3D point data cloud created from the received radar signals.

Assumptions about the required minimum number of frames for obtaining the objectives of the present invention could be made. According to the Nyquist-Shanning sampling theorem, a periodic signal must be sampled at more than twice the highest frequency component of the signal, which can be mathematically written as $f_s > 2f_{max}$, where $f_s$ represent the number of frames per second and $f_{max}$ represent the highest frequency that is desirable to detect. If, as an example, it is desired to estimate a respiration rate of 30 rpm, the theoretical minimum number of frames per second would be 2. If it is desired to estimate a heart rate of 150 bpm, the theoretical minimum number of frames per second would be 6.

This is the theoretical minimum number of frames needed, and any increase in the frame frequency would improve the resolution and hence the results.

A first step of the preferred method of the invention is noise filtering of the raw data to remove irrelevant information from the signal. This step is illustrated as item 3 in figure 1. As further explained below, this is an optional step.

Various types of noise reduction techniques can be used to filter various types of noises from the frames. The goal of applying these filters is to improve the predictive quality of the repository rate model. The filtering process may include identifying and removing points with velocities that significantly exceeds the normal chest expansion speed during inspiration and expiration. This can be done by measuring and calculating the mean chest expansion velocity and the standard deviation. Points with velocities that lies within, e.g., three standard deviations $\sigma$ of the mean value $\mu$ may be treated as noise and removed. That is all points with a velocity $V > \mu_V + 3 * \sigma_V$, where

$$\sigma_V = \frac{\sqrt{\sum (v_i - \mu_V)^2}}{N} \qquad (1)$$

$$\mu_V = \frac{\sum v_i}{N} \qquad (2)$$

where $\sigma_V$ is the standard deviation of the velocity, $v_i$ is velocity of point $i$, $\mu_V$ is the mean of the velocities and $N$ is the number of points.

In situations where static objects are making movements that falls within a frequency band that may interfere with the movements that are desirable to detect (e.g., respiration and heartbeat), such as a spinning fan, a mechanical pump, ventilator machines, patient monitors etc., the above-described way of filtering could remove such interfering objects from the point cloud if their point-velocities significantly exceeds that of, e.g., a normal chest expansion. If, however, the velocities are in the same frequency band and with similar velocities as the objects that are desirable to detect, the points need to be filtered in other ways. Such other filtering techniques could include putting constraints on the size of the point cloud, requiring that the object fits into similar measures as a body to be recognized as a living creature, e.g., a human being, requiring that the object has moved some distance during a defined time-interval, etc.

In situations where another living creature is in detection range of the radar, and the frequency and velocity overlap with the values from a human, constraints about the size and shape of the object could be applied to remove these points from the point cloud.

Other useful statistical noise reduction techniques may also be used, such as interquartile range filtering, kernel estimation, and smoothing. All of these techniques are well known per se.

The noise filtering step may improve the efficiency and quality of the subsequent steps, but is considered a non-critical step, as acceptable results for some applications could be achieved by skipping this step, given that the input point cloud data does not contain large amounts of noise. This is dependent on the environment that the radar will operate in. Depending on the data quality, the object identification step described below may sufficiently exclude erroneous data points.

The points in the 3D point cloud may originate from different objects and noise sources within the scene. In order to estimate the respiration rate of a single or even multiple humans in the scene simultaneously and with sufficient accuracy, the method must be able to separate and assign the points into different groups. The groups may represent noise or objects of interest. The identified objects must then be tracked across consecutive frames in order to isolate and extract their continuously varying movement information.

According to the invention object identification techniques (shown as item 4 in figure 1) are applied in order to segment 3D point clouds into different objects, such as persons. Given a set of points, the objective of the object identification process is to group points by spatial proximity into homogeneous regions. These isolated regions, by their characteristics, represent different objects in the scene. Grouped points that are identified to derive from chest expansions may be isolated and used as input to the repository rate model. This approach allows for separation and monitoring multiple targets, such as patients within the scene.

In a preferred embodiment the method of the invention implements the use of DBSCAN as object identification algorithm for this step. This alternative is illustrated as item 4A in figure 1. DBSCAN is a widely used object identification algorithm that is known per se, and it has several advantages which makes it a good choice for this grouping problem:

DBSCAN is a density-based grouping algorithm where it is only needed to specify the minimum number of points $N$ in each group and the maximum distance $\varepsilon$ between neighbouring samples. This algorithm is, e.g., described in Schubert, Erich, et al. "DBSCAN revisited, revisited: why and how you should (still) use DBSCAN." ACM Transactions on Database Systems (TODS) 42.3 (2017): 1-21.

The number of groups can be arbitrary large and do not need to be predefined. This allows for simultaneous tracking of one or several persons in the room without prior knowledge of how many persons are in the room.

The algorithm can identify less obvious noise based on the positions of the points.

DBSCAN do not require the group to have a specific geometry. This also makes It more robust to different lying and sitting positions of the person associated with the group.

The algorithm of DBSCAN can be summarized as follow:

1. A random point $x_i$ is picked and defined as a core point.

2. If $x_i$ has at least $N$ neighbouring points within a radius $\varepsilon$, $x_i$ is defined to belong to a group and is defined as a new core point.

3. Step 2. is performed recursively for all neighbours of $x_i$ within the radius $\varepsilon$ and subsequently for all the neighbours' neighbours that are within the radius $\varepsilon$. The group is expanded until there no longer exist any point within the radius $\varepsilon$ to any of the core points.

4. Step 3. stops when there no longer exist any new neighbours within the radius $\varepsilon$. A new random point $x_i$ is then selected and the algorithm restarts at step 1. This is continued until there are no new points left.

5. At the last step of the algorithm, all points with fewer than $N$ neighbours will be added to the group with the closest core point within the radius $\varepsilon$. Points with no core points within the radius $\varepsilon$ are classified as noise.

Figure 4 illustrates the DBSCAN algorithm with two groups and one noise point. Around each point a circle with radius $\varepsilon$ is drawn around each of the points. For the points 10a, 10b and 10c, all of the points fall within the radius $\varepsilon$ of each other, while none of the other points wall within this radius. Consequently, these points clearly belong to the same group.

Points 11a, 11b, 11c also fall within each other's radius $\varepsilon$ and clearly belong to the same group. Point 11d falls outside of the radius $\varepsilon$ of points 11a and 11b, but within the radius $\varepsilon$ of point 11c. Consequently, point 11d is also defined as a part of the same group.

Point 12 does not fall within the radius $\varepsilon$ of any of the other points, and hence point 12 does not belong to any of the groups and is defined as noise.

Other grouping algorithms may alternatively be used to group the 3D point-cloud data, as illustrated by item 4B in figure 1. Examples of other known per se algorithms are:

- OPTICS (Ankerst, Mihael, Markus M. Breunig, Hans-Peter Kriegel, and Jörg Sander. "OPTICS: Ordering points to identify the clustering structure." ACM SIGMOID Record 28, no. 2 (1999): 49-60.),
- HDBSCAN (McInnes et al, (2017), hdbscan: Hierarchical density-based clustering, Journal of Open Source Software, 2(11), 205, doi:10.21105/joss.00205),
- Mean-Shift Clustering (Dorin Comaniciu and Peter Meer, "Mean Shift: A robust approach toward feature space analysis". IEEE Transactions on Pattern Analysis and Machine Intelligence. 2002. pp. 603-619.), and

- Affinity-Propagation Clustering (Brendan J. Frey and Delbert Dueck, "Clustering by Passing Messages Between Data Points", Science Feb. 2007).

Important characteristics of theses grouping approaches include the ability to detect the number of groups automatically and find outlier points, i.e., points with an insufficient number of neighbours in proximity. These algorithms are likely to produce comparable results as the DBSCAN algorithm, albeit with variations in their characteristics regarding complexity, speed and memory usage.

After the at least one object has been identified according to the above step, the objects must be tracked in order to follow the movement of the person(s).

Grouped points are represented with a minimum bounding box, which is the smallest box that contains the points identified as belonging to one group in the 3D space. Instead of a box, other shapes can be chosen, such as a sphere, a polyhedron or an irregularly shaped 3D boundary. A box, however, is a simple geometric shape that can be represented by few parameters, which requires limited computational power. Figure 3a shows a 3D point cloud where some points have been found to belong to the same object and have been assigned a minimum bounding box that just encloses all the point of the object.

Objects are then tracked across frames by testing for overlapping geometries. In other words, grouped points found in two consecutive frames along the time axis can be determined to originate from the same object if the minimum bounding box from the grouped points overlap across the two frames. The method for object tracking is summarized as follows:

   i. Calculate the minimum bounding box for each group of points in a frame.
   ii. Determine if any of the minimum bounding boxes found in the current frame overlap with any of the minimum bounding box from the previous frame.
   iii. Update the state table that keeps track of objects and temporal movement variations.

      a. Update the geometry of objects that overlaps between the current and previous frame with the new minimum bounding box and track the change in velocity.
      b. Add any group that does not overlap with any minimum bounding box from the previous frame as new objects.
      c. Remove any object from the previous frame that does not overlap with any of group in the current frame.

The method assumes that the update frequency of the radar is sufficiently high so that the geometry of the grouped points that originate from the same object will always overlap across consecutive frames even if the object moves randomly at normal walking speed. This means that the frame rate has to be sufficient high that a person is not capable of moving a longer distance than the width of the minimum bounding box between two consecutive frames. For tracking human beings, a frame rate of 20 fps is a typical choice.

This method makes it possible to dynamically isolate and monitor objects in the room and detect if objects moves, enters or leaves the room. The geometrical properties of the minimum bounding boxes can also be leveraged in combination of the tracking procedure to keep objects from merging at close distances.

Next step of the method of the invention is to apply an aggregate function. Aggregation functions are well known per se (see for example https://www.investopedia.com/terms/a/aggregate-function.asp ), but according to the invention it is used to group and form the grouped velocity data into a single summary signal that captures all the relevant movements of the group.

The large movements (chest expansion) that occurs during respiration will increase the size of the group, and thus also the sum of magnitude points will increase. Many of these points will not necessarily have a high magnitude, and thus a summation of the points is a good aggregation function for respiration rate estimation. Other examples of aggregation functions include average, median, maximum, minimum and range. Average and median use the average of all items and the median item respectively to obtain the single summary signal, maximum and minimum uses the item with maximum and minimum value respectively while range uses the difference between the maximum and the minimum value.

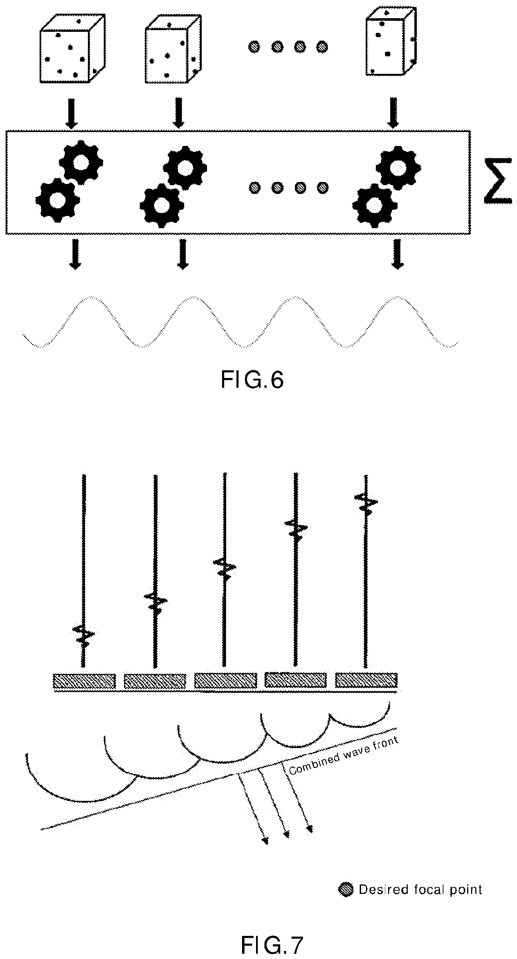

Figure 6 illustrates the aggregate function. At the top row of the figure are bounding boxes of several consecutive frames shown. Both the shape and size of the boxes may vary slightly from frame to frame. In this step, however, the important parameter is the combined speed, both in absolute value and direction, the important factor. Through the aggregate function, which is illustrated by the cog wheels in the second row, a graph of the combined velocity of the bounding box in each frame is plotted. In this example, the graph is a sinus curve, which would be typical when respiration is tracked. From the graph both the frequency and amplitude of the respiration can be deducted.

The next step of the method of the invention is optional but may greatly enhance the signal quality. This step is a dynamic focus adjustment, illustrated by item 5 in figure 1. In this step the focus parameters of the radar sensor are focused dynamically in order to maximize signal quality of the subsequent point-cloud dataframe. When a set of points, such as objects 10 and 11 in figure 4, are identified to belong to human beings, the focus area of the radar is centred to the centroid, i.e., the arithmetic mean position of all the points, of the bounding box.

If the system detects that one of the groups has a respiration or heartbeat outside of the normal range, the radar can focus mainly on that group to increase the resolution and improve data quality. Hence, a beginning critical condition, such as a heart attack, can be detected at an early stage.

In order to detect new persons arriving onto the space, such as a patient room, the radar will at regular intervals scan the whole space in search for new groups. When a new group is detected, the system will include that group in the focused areas.

If a person leaves the space, the group will disappear, and the system can exclude the area that that group occupied from the focused areas.

The system may also be set up to determine a likely orientation of the subject. When breathing the characteristics of the points in the group will be different if the subject, such as a person, is facing the radar or having its side towards the radar. The characteristics will again be different if the person is lying down. If the person has the back towards the radar, there will be a greater spread of points moving than if the person is facing the radar with the chest. The same techniques as explained below may be used in determining the orientation of the subject.

It is possible to equip persons with a radar reflector that when detected tells the system that this person is a caretaker. The system can then exclude the group that that person represents from the focused areas or reduce the resolution of 3D data points for that group. Alternatively, patients can be equipped with a radar reflector to tell the system to increase the resolution for the group representing that person. Visitors may also be equipped with such radar reflectors.

The focus area, or a part of the focus area, will shift in space as a person moves. This movement can be detected in various ways, such as by monitoring velocity vectors of the data points of the group or letting the focus area cover a limited portion outside the group.

By dynamically adjusting the focus area per frame to the bounding box centroids it is possible to track objects within the scene and increase signal quality. Multiple targets may also be tracked by rapidly shifting focus between them. An alternative approach to track multiple targets is expanding the focus area to cover several groups. This will however be at a possible cost of decreased spatial resolution and decreased signal-to-noise ratio. This way of dynamically focusing the radar could be achieved either through software or hardware.

The radar that is contemplated to be used in in connection with the method of the present invention is preferably of a type that can detect both regular Doppler points and micro-Doppler points. Regular Doppler points are frequency shifts of large motion movements of a target relative to the detecting radar. In addition to the large motions, the target undergoes micro-motion dynamics such as rotations and vibrations. These micro-motion dynamics induce Doppler modulations on the returned Doppler signal and is referred to as the micro-Doppler effect. A further explanation of this phenomenon can be found at https://www.researchgate.net/publication/3003947_Micro-Doppler_Effect in_Radar_Phenomenon_Model and Simulation Study. The points generated by the micro-Doppler effect are especially suited for detecting body micromotions such as respiration and heartbeat.

The radar contemplated to be used has the ability to adjust the sensitivity towards both regular Doppler points and micro-Doppler points while the radar is operational. Adjustment of, e.g., Doppler sensitivity will not affect the micro-Doppler points in itself, but the maximum number of possible Doppler and micro-Doppler points is limited by available processing power.

Focusing through software could be achieved by using a combination of both micro-Doppler and regular Doppler points. Regular Doppler points are used to track a subject's movements by creating a bounding box which defines the location of the subject. When the radar detects a stop in movement of the bounding box, the coordinates of the last known location of the bounding box is saved. The sensitivities of the regular and micro-Doppler points are then adjusted to be more sensitive towards micro-doppler points. This enables processing of a higher number of the points that are used for detecting respiration and heart rate and reduces the number of large motion points, which could introduce noise in the signals used by the estimation methods.

After adjusting the sensitivity, points outside the last known location of the bounding box are filtered out, leaving only points belonging to the subject. This step further removes micro-Doppler noise from the surrounding area, resulting in a cleaner signal that would increase the performance of the estimation methods. The remaining points after filtering are then used for estimating respiration and heart rate. Consequently, a higher resolution is achieved of the respiration and heart-beat monitoring and at the same time noise is reduced.

Even though the focus of the system is on the data points belonging to the group of micro-Doppler points, the system of the invention will still monitor enough regular Doppler points to be able to determine if the subject has moved as a whole, i.e., the Doppler shifts of several data points is larger than the range expected by the micro-Doppler shifts. When it is determined that the subject is moving, the system may adjust the sensitivity to be more sensitive towards the larger Doppler shifts in order to track the movement of the subject.

The above process could also be applied to multiple subjects allowing for focusing on multiple subjects at the same time. This would of course mean that as there is a maximum number of points that can be processed by the system per second, the resolution for each subject will be reduced. However, depending on capacity of the processor, the resolution may still be sufficient to monitor two or more subjects at the same time.

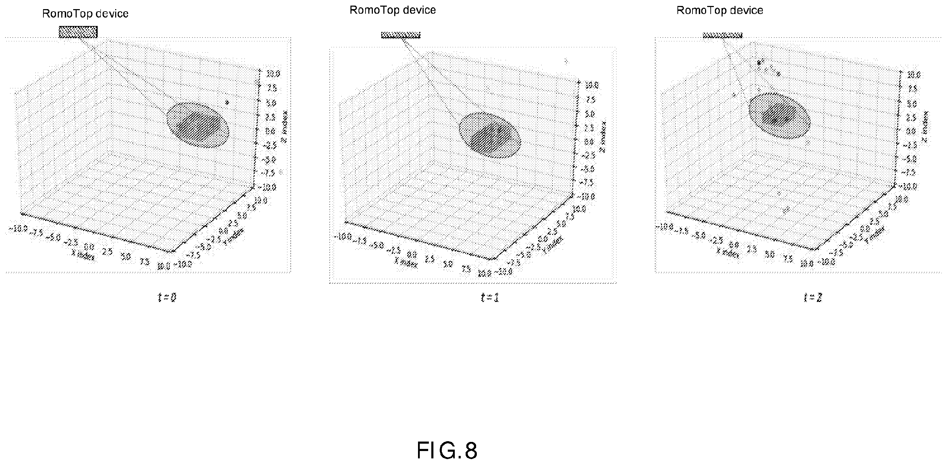

Focusing through software could be achieved by, but is not limited to, adjusting the time delays of the elements of either the transmitter (TX) or the receiver (RX). Figure 7 shows dynamic focusing with time-delays applied at the TX. The applied time-delays causes the elements to transmit waves at different times, sending the combined wave front in the direction of the desired focal point. Figure 8 shows an illustration of how dynamic focusing through software could be performed on the point cloud. From figure 8a, through figure 8b, to figure 8c, the bounding box has moved towards the radar, hence the focus of the radar shifts to focus at a closed distance.

Another way of achieving a dynamic adjustment of the focus is through hardware. The radar could be mounted onto some controllable hardware, such as but not limited to a robotic arm, gyroscope, servomotor, hydraulic actuators, vacuum actuators and/or other mechanical actuators. By controlling the angle of the actuator, the radar beam could be focused in the direction of the point cloud.

From this point onwards, the objects of the invention can be realized in two different ways, illustrated by the two branches 7A and 7B, respectively, in figure 1, or they can be realized by performing the steps of both branches 7A and 7B on the dataset, after which the results are combined in a step 8. This will be explained below.

First the branch 7A will be explained.

The branch 7A is a frequency extraction, which is based on traditional methods. Traditional signal processing methods can be used to detect the respiration with satisfactory results. The algorithm preferably used in the present invention relies on a technique called multitaper to find dominant frequencies by estimating the spectral density of the signal. Multitaper is one of multiple methods to estimate the spectral density of a random signal from a sequence of time samples of the given random signal. Other examples of techniques for spectral density estimation are least-squares spectral analysis, non-uniform discrete Fourier transform, singular spectrum analysis, short time Fourier transform, and critical filter.

The estimated spectral density of a signal describes the distribution of power into the frequency components and is used to detect periodicities in the signal. Periodicities are found by seeking intensity peaks at different generated frequency components. This includes the peaks from the periodic movement of respiration and heartbeats. The respiration rate and heart rate can be estimated by seeking the dominant frequency components in the velocity signal within non-overlapping frequency ranges. Spectral density estimation techniques may also be performed on time samples from the velocity signal from multiple groups independently and allow tracking of the respiration rate and heart rate from multiple humans in the scene simultaneously. The frequency range of respiration rate and heart rate will be different, and there are likely to be two large periodic components in the signal: a strong lower frequency component due to respiration (6 to 25 bpm) and a strong higher frequency component due to heart beats (60 to 180 bpm).

The detrending and filtering step 7A2 is performed on the group signal in order to maximize the quality of the spectral density estimate. Trends are in this case not considered to be a property of the interesting signal and can be removed by subtracting the signal with its mean value.

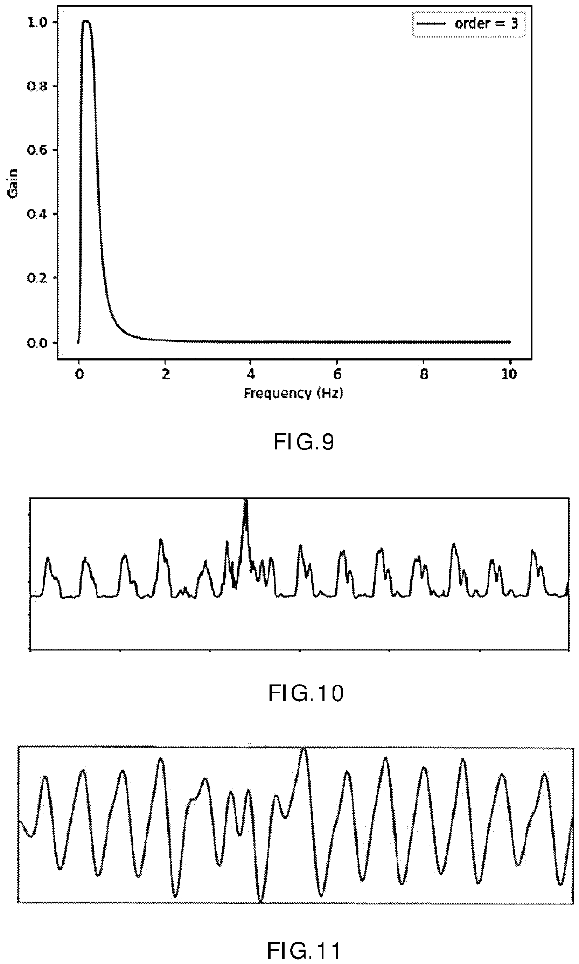

Detrending reduces the signal component at low frequencies. A low-pass and high-pass Butterworth filter are then applied in order to smooth out noise from the signal, remove unwanted frequency components and separate the respiration signal and the heartbeat signal before estimating the spectral densities. Figure 10 shows an exemplary input respiration signal before filtering. Figure 9 shows an example of a Butterworth filter adapted to filter respiration signals. As a normal breathing pattern has a frequency of about 0,2 - 0,5 Hz, the filter has a high gain around this frequency.

Figure 11 shows the signal after being filtered with the filter of figure 9. As can be seen here, the breathing pattern has become clearly visible.

The next step of branch 7A is to perform multitaper, as illustrated by step 7A2. A background on how this method in general works can be found in:

Babadi, Behtash, and Emery N. Brown. "A review of multitaper spectral analysis." IEEE Transactions on Biomedical Engineering 61.5 (2014): 1555-1564.

One of the simplest techniques to estimate spectral density is the periodogram. The periodogram is the frequency spectrum of a set of time signals and can be defined as the Fourier transform of the autocorrelation. If it is assumed that a sample of size $N$ of the random signal $x_k$ for $k$ = 0,1, 2, ... is given, an approach to obtain a nonparametric estimate of the autocovariance sequence $s_k$ is the method of moments, where the estimate can be based on the sample mean and sample autocovariance

$$\hat{\mu} = \frac{1}{N} \sum_{i=0}^{N-1} x_i \qquad (3)$$

where $\hat{\mu}$ denotes the sample mean, and

$$\hat{s}_k = \frac{1}{N} \sum_{i=0}^{N-k-1} (x_i - \hat{\mu})(x_{i+k} - \hat{\mu}) \qquad (4)$$

Taking the Fourier transform of the estimates above gives us the periodogram:

$$\hat{S}(f) = \Delta \sum_{k=-N+1}^{N-1} \hat{s}_k e^{-i2\pi k f \Delta} = \frac{\Delta}{N} \left| \sum_{k=0}^{N-1} x_k e^{-i2\pi k f \Delta} \right|^2 \qquad (5)$$

where $\hat{S}(f)$ is an estimate of the true power spectral density $S(f)$ of a random signal $x_k$, $\Delta$ denotes the sampling interval, $f$ denotes the frequency and the Fourier transform of $x_k$ is defined as

$$X(f) := \Delta \sum_{k=0}^{N-1} x_k e^{-i2\pi k f \Delta}. \qquad )6)$$

The multitaper method is based on the periodogram but improves further on it by using multiple windows to form independent estimates of the spectral density. If we let $h_k^{(1)}, h_k^{(2)}, \ldots, h_k^{(L)}$ be a set of tapers, the multitaper (mt) spectral estimate for $L$ windows is given by

$$\hat{S}^{\mathrm{mt}}(f) := \frac{1}{L} \sum_{i=1}^{L} \hat{S}^{(i)}(f) \qquad (7)$$

where

$$\hat{S}^{(i)}(f) := \Delta \left| \sum_{k=0}^{N-1} h_k^{(i)} x_k e^{-i2\pi k f \Delta} \right|^2. \qquad (8)$$

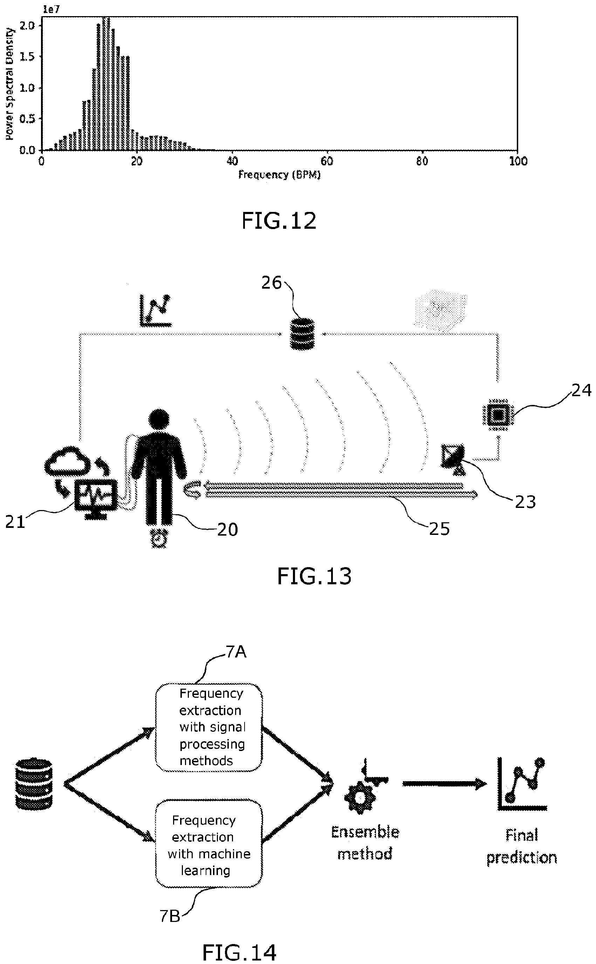

The approach of combining the results from multiple windows reduces the variance of the final spectral density estimate.
An example of a multitaper spectral density estimate derived from a test subject with a respiration rate of 13 breaths per minute is shown in figure 12.
Next in the method of the invention, the second branch 7B of figure 1 will be described. In this branch, the system uses machine learning to optimize the respiration and/or heartbeat signals. The application of machine learning to solve computational challenges has emerged rapidly over the last years due to the combination of improvements in algorithms and accessibility of computing power. In contrast to programming an explicit solution, machine learning trains a model to solve the problem by feeding an algorithm with historical data. This allows the model to learn complex patterns for modelling the data, which often would be infeasible to program explicitly. For the challenge of modelling respiration based on 3D point cloud data, is needed to collect labelled data. As illustrated in figure 13, a patient 20 is coupled to a patient monitor 21. The radar 22, which is coupled to a processor 23 transmits and receive radar data 25. The processor 24 stores processed data in a training database 26. Data from the patient monitor 21 is also transferred to the database 26. According to the invention this collection of large amounts of labelled data from the patient monitor 21 is synchronized against the radar data. The data is recorded under a range of demanding conditions (such as long distances, introduction of noise).
The synchronized dataset is then used to train the model to extract respiration directly from the 3D point cloud input data. The application of machine learning for this purpose improves the quality and robustness of the detections, especially under challenging conditions. The main contribution of using a machine learning method for this purpose is that it can learn to model the signal under a wide range of otherwise challenging conditions, provided it is trained with data that represents similar conditions that it is trained to model.

A regression analysis, as illustrated by item 7B1 in figure 1, is a set of statistical processes of estimating relationships between an outcome variable and one or more features. The desired output of the model is detection of respiration; thus, regression methods are a good fit.

There are a wide range of well-known regression methods that can use the movement information of groups to predict respiration. An overview of these can be found in the following article: https://www.listendata.com/2018/03/regressionanalysis.html.

Next gradient boosting, as illustrated by item 7B1A is used to produce a prediction model. Gradient boosting is a machine learning technique for regression and classification problems, which produces a prediction model in the form of an ensemble of weak prediction models, typically decision trees. It builds the model in a stage-wise fashion, and it generalizes them by allowing optimization of an arbitrary differentiable loss function.

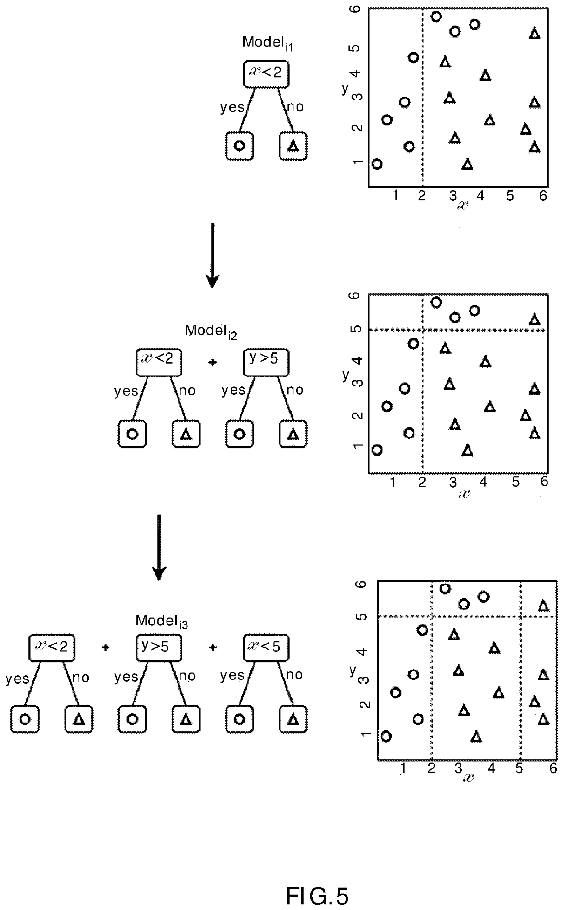

Gradient Boosting is well-described in J. Friedman. Greedy function approximation: a gradient boosting machine. Annals of Statistics, 29(5):1189-1232, 2001. Figure 5 illustrates of how gradient boosting works with decision trees.

In the first iteration shown in figure 5, a single decision tree is built to fit the training data. In this iteration data points that readily can be identified as good data (in the example having an x value below 2) is classified into one group, denoted by "yes" and the rest of the datapoints that cannot readily be classified, are sorted into another group, denoted by "no".

Next, in a 2$^{nd}$ iteration a new decision tree is built, focusing on the part of the training data that the previous model made a bad prediction on, i.e., in the "no" group. In the example the focus is here on datapoints that readily can be identified as having a y value larger than 5.

The two models of the previous iterations are then combined into a single ensemble model, and the second iteration step is executed again.

In the 3$^{rd}$ iteration, datapoints classified as bad data (labelled with "no") in the second iteration are sorted again. In the example with the criterion x smaller than 5. This creates a third decision tree.

The process is repeated, where each new decision tree will attempt to correct the errors made by the combined ensemble model of previous decision trees.

The process is repeated until it starts overfitting, or the sum of the error residuals become constant.

Several Gradient Boosting algorithms are publicly available as open source implementations:

- LightGBM (Guolin Ke, Qi Meng, Thomas Finley, Taifeng Wang, Wei Chen, Weidong Ma, Qiwei Ye, and Tie-Yan Liu. 2017. LightGBM: a highly efficient gradient boosting decision tree. In Proceedings of the 31st International Conference on Neural Information Processing Systems (NIPS'17). Curran Associates Inc., Red Hook, NY, USA, 3149-3157),

- XGBoost (Tianqi Chen and Carlos Guestrin. XGBoost: A Scalable Decision Tree Boosting System. In 22nd SIGKDD Conference on Knowledge Discovery and Data Mining, 2016.),

- AdaBoost (Yoav Freund and Robert E. Schapire. A decision-theoretic generalization of on-line learning and an application to boosting. Journal of Computer and System Sciences, 55(1):119-139, August 1997.) and

- CatBoost (orenkova, Liudmila, et al. "CatBoost: Unbiased boosting with categorical features." Advances in neural information processing systems. 2018.).

Item 7A1B in figure 1 illustrates these alternatives in the method of the invention.

In addition to gradient boosting, several alternative methods to solve the regression problem exists. These include Random Forest Regression, Artificial Neural Network regression, K-nearest neighbour-regression, linear regression, logistic regression, ridge regression, lasso regression, polynomial regression, stepwise regression, ElasticNet regression, principal component regression, binomial regression, binary regression, Poisson regression, support vector machines (SVM), naive Bayes and expectation maximization (EM) algorithm.

Random Forest Regression is an ensemble method that operate by constructing a multitude of decision trees at training time. However, Random Forest Regression averages the predictions from all the individual regression trees at prediction time. This is illustrated by item 7B in figure 1.

Artificial Neural Networks (ANN) are composed of individual "neurons", which has one or more input(s) and produce a single output. The neurons are organized in several layers, and the outputs from the final layer accomplish the regression task. Such ANN could be composed of linear layers, convolutional layers (CNN) and/or recurrent layers (RNN). These networks can be used for regression if the final output node of the network is configured to produce a single continuous value.

A final step of the method of the invention in reaching an optimized signal by the steps of branch 7B is to use an algorithm called CatBoost, as illustrated by item 7B2 in figure 1.

CatBoost is a state-of-the-art machine learning algorithm based on Gradient Boosting. CatBoost introduces two critical algorithmic advances compared to existing solutions: the implementation of ordered boosting, a permutation-driven alternative to the classic algorithm, and an innovative algorithm for processing categorical features. CatBoost is also significantly faster than existing methods, making it highly suitable for latency-critical tasks. This is especially true when running on a Graphical Processing Unit (GPU), which is supported out of the box.

In the next step, illustrated by 8 in figure 1, is an ensemble learning technique used. In machine learning, ensemble learning describes the use of multiple learning algorithms to obtain better predictive performance than could be obtained from any of the constituent learning algorithms alone. This is generally described in:

Opitz, David, and Richard Maclin. "Popular ensemble methods: An empirical study." Journal of artificial intelligence research 11 (1999): 169-198

In a further embodiment, input from further radars can be combined with the data from the first radar. More specifically, similar processes as illustrated by the branches 7A and 7B, and explained above, are performed on the data from the further radars. The processed data from these radars are then used as input in the ensemble step in addition to the data from the first radar and hence subjected to the same ensemble learning method as described herein. This may further improve the reliability of the results. This could be particularly useful in scenarios where multiple radars are installed with an overlapping coverage area. The output from multiple radars would provide a larger confidence in the final predictions. Figure 14 illustrates that the outputs of branches 7A and 7B, according to the invention, could be combined and used as input to a second machine learning model, chosen between the methods described in 7B, in order to reduce the overall error of the modelled respiration signal. This technique will learn to weight the two input signals differently to produce the final output signal.

In the following will be described experimental results using the method of the invention. Here will be described dataflow during the experiments, how the different experiments are set up and lastly, will be presented the results from the improved algorithm of the invention.

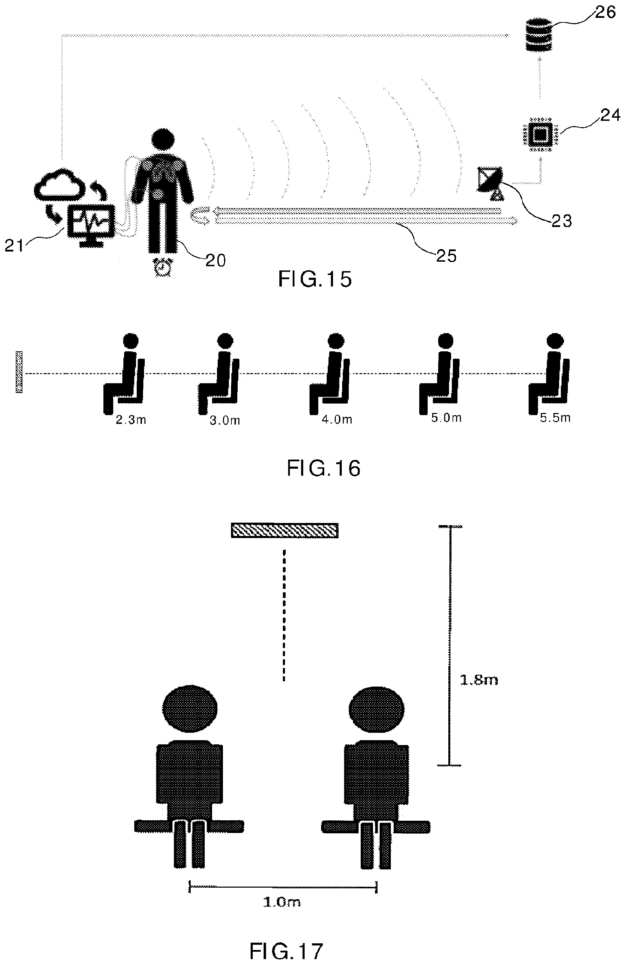

Figure 15 shows the experimental setup and the dataflow during the experiments. The setup is similar to the setup of figure 13

In figure 15, a subject 20 was connected to a patient monitor 21 called Recobro Vigile™ through a 5-lead electrode setup. This patient monitor is a state-of-the art medical device that can be used to measure vital signs such as heart rate, respiration rate. The output of this patient monitor is used as reference when comparing the proposed method of the invention to existing methods. The radar 23 records 3D pulse-Doppler datapoints that are processed on a sensor CPU 24 by the proposed algorithm of the invention, as described above. Both the output from the CPU 24 and the patient monitor 21 are stored in a database 26. Here the output from the method of the invention is compared to the vital signs from the patient monitor 21.

Any type of high-quality equipment that can detect respiration or heartbeat rate can be used instead of the specific patient monitor mentioned above.

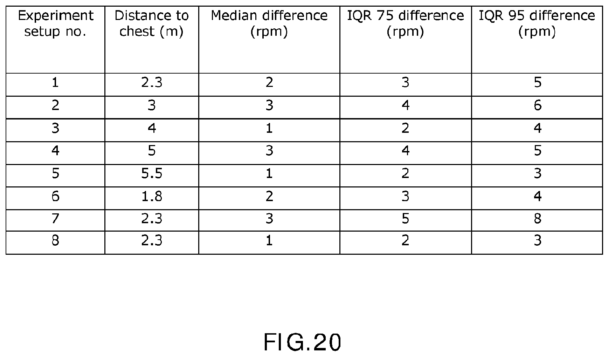

In a first experiment, a test subject was placed sitting in a chair, with the chest facing the radar 23. The same experiment was performed at all the distances illustrated in figure 16, namely 23,3m; 3,0m; 4,0m; 5,0m; and 5,5m distance from the radar 23 to the chest of the person. The results of this experiment are labelled 1-5 in the table of figure 20.

In a second experiment, illustrated in figure 17, one test subject and an additional person were sitting next to another with a separation of 1m. The distance to the radar was 1,8m, with the radar mounted in the ceiling vertically above the two persons. The results of this experiment are labelled as 6 in the table of figure 20.

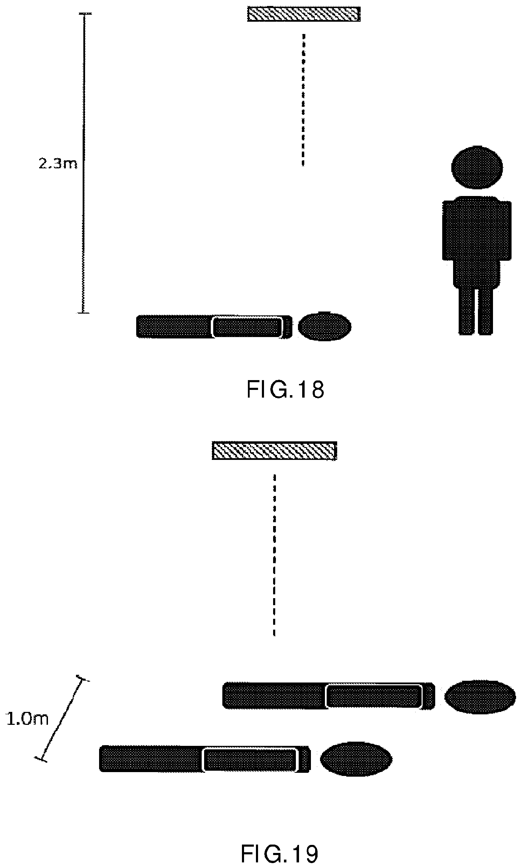

In a third experiment, illustrated in figure 18, a test subject was lying on a bed with the radar vertically above at a distance of 2,3m from the chest of the subject. Another subject was walking around in the room next to the test subject. The results of this experiment are labelled as 7 in the table of figure 20.

In a fourth experiment, illustrated in figure 19, a test subject was lying in a bed with the radar 2,3m vertically above the chest. Another person was lying in another bed at the same elevation level 1m from the test subject. The results of this experiment are labelled as 8 in the table of figure 20.

For all experiments were used a 60 GHz mm-wave radar.

The table of figure 20 shows in the first column the labels of the experiments above. The second column indicates the distances from the radar to the chest of the test subject. The third gives the median difference in respirations per minute between the respiration measurements done according to the present invention and the accurate measurements done by the patient monitor 21. The fourth column gives the interquartile range (IOR) at the 75th percentile between the measurements done according to the present invention and the accurate measurements done by the patient monitor 21, while the final column gives the same at the 95th percentile.

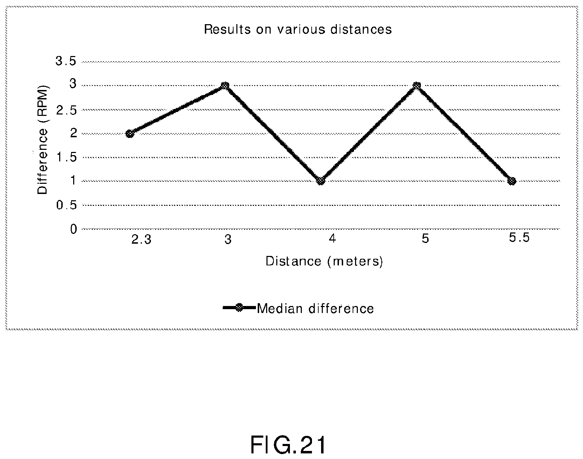

As is evident from the results shown in figure 20, the method of the invention performs very well. The difference between the actual respiration rate and the respiration rate determined by the system of the present invention is low, and it is fairly consistent. The distance between the radar and the test subject does not seem to have any significance. The ability to model respiration at longer distances is shown in figure 20, achieving good results on distances up to 5.5 meters. The minor differences are attributed to variations in the behaviour of the test subjects in each experiment. Consequently, the method of the invention enables respiration detection and estimation for different scenarios, including single- and multi-person scenarios, under various conditions. The method is robust against noise and gives the ability to identify and track objects confidently at various distances. Figure 21 shows the difference in respiration rate versus distance as a graph.

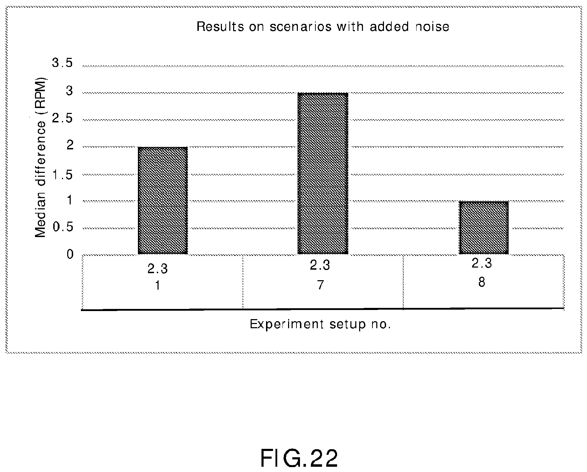

Figure 22 shows the ability of the system of the invention to model respiration under noisy conditions. As can be seen from this diagram, there are no substantial differences in the results achieved in experiments with added "noise" as described in experiment setups 7 and 8 above, compared to the results of experiment 1, which is without noise added. The minor differences are attributed to variations in the behaviour of the test subjects in each experiment.

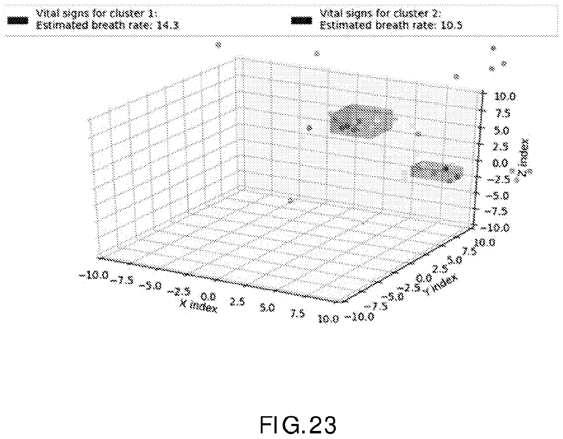

Figure 23 shows a 3D point cloud with bounding boxes arranged around the points that have been identified to belong to specific subjects according to experiment setup no. 8. The ability of the method to perform multi-person detection and respiration rate estimation is shown here. The method of the invention can both capture multiple persons, as illustrated by the bounding boxes, and the method is also able to estimate respiration rates, as shown by the values assigned to each of the boxes (at the top of the figure).

Prediction on the optimized signal has in experiments proven to provide more accurate respiration values, and the ability to do respiration detection on longer distances, than has been previously possible.

**Claims**

1. A method of optimizing a dataset from a phased-array radar to detect movement of subjects (20) within a coverage area of the radar, wherein said radar creates a 3D point-cloud dataset (2), said dataset (2) being fed into an object identification algorithm (4) to create at least one group of datapoints (10a, b, c; 11a, b, c) **characterized by** their proximity and their pattern of movement, defining that said at least one group (10a, b, c; 11a, b, c) belongs to a single subject (20) and tracking movement of datapoints of said group (10a, b, c; 11a, b, c); wherein after said point grouping, defining a minimum bounding shape, such as a box, around said at least one group (10a, b, c; 11a, b, c) and tracking said minimum bounding shape across frames of 3D point clouds taken at different consecutive times, wherein said tracking involves the following steps:

   i. calculate the minimum bounding shape for each group of points in a frame,
   ii. determine if any of the minimum bounding shapes found in a current frame overlap with any of the minimum bounding shapes from a previous frame,
   iii. keeping track of overlapping objects and temporal movement variations, wherein said

   tracking of said overlapping objects comprises:

   a. updating a geometry of objects that overlaps between said current and said previous frames with a new minimum bounding shape and track a change in velocity of said minimum bounding shape,
   b. add any group that does not overlap with any minimum bounding shapes from said previous frame as a new object, and
   c. remove any object from said previous frame that does not overlap with any group in said current frame.

2. The method of claim 1, wherein the movement of subjects is micro body movement of subjects due to heartbeat or respiration and further comprising:
   aggregating (5) the datapoints (10a, b, c; 11a, b, c) of said group to form a single velocity signal (9) and extracting a heart rate or a respiration rate from the single velocity signal (9) of said group (10a, b, c; 11a, b, c), wherein a focus adjustment of said radar (23) is done by dynamically focusing on a centroid of said at least one group (10a, b, c; 11a, b, c).

3. The method of claim 2, wherein said aggregation of datapoints comprises a summation of points related to large object movements.

4. The method of claim 2 or claim 3, wherein said aggregation (5) comprises a single summary velocity signal based on averaging a velocity of each point of said group (10a, b, c; 11a, b, c), taking the median velocity of said group (10a, b, c; 11a, b, c), determining the maximum velocity of said group, determining the minimum velocity of said group (10a, b, c; 11a, b, c) or determining the range of velocities of said group (10a, b, c; 11a, b, c) and use the range as said single summary velocity signal (9).

5. The method of any of claims 2 to 4, wherein the phased-array radar (23) transmits at a frequency of 60 - 81 GHz, preferably between 60 and 64 GHz and most preferably around 60 GHz.

6. The method of any of claims 2 to 5, wherein said 3D point cloud is subjected to noise filtering (3) before being fed into said identification algorithm (4).

**Patentansprüche**

1. Verfahren zum Optimieren eines Datensatzes von einem Phased-Array-Radar zum Erkennen von Bewegung von Subjekten (20) innerhalb eines Abdeckungsbereichs des Radars, wobei das Radar einen 3D-Punktwolkendatensatz (2) erstellt, wobei der Datensatz (2) in einen Objektidentifikationsalgorithmus (4) eingespeist wird, um mindestens eine Gruppe von Datenpunkten (10a, b, c; 11a, b, c) zu erstellen, **gekennzeichnet durch,** ihre Nähe und ihr Bewegungsmuster, die definieren, dass die mindestens eine Gruppe (10a, b, c; 11a, b, c) zu einem einzelnen Subjekt (20) gehört, und die Bewegung von Datenpunkten der Gruppe (10a, b, c; 11a, b, c) verfolgt wird; wobei nach dem Punktgruppieren eine Minimum-Bounding-Form, wie eine Box, um die mindestens eine Gruppe (10a, b, c; 11a, b, c) definiert wird und die Minimum-Bounding-Form über Rahmen von 3D-Punktwolken hinweg verfolgt wird, die zu unterschiedlichen aufeinanderfolgenden Zeitpunkten aufgenommen werden, wobei das Verfolgen die folgenden Schritte einschließt:

   i. Berechnen der Minimum-Bounding-Form für jede Gruppe von Punkten in einem Rahmen,
   ii. Bestimmen, ob eine beliebige der Minimum-Bounding-Formen, die in einem aktuellen Rahmen gefunden wird, mit einer beliebigen der Minimum-Bounding-Formen eines vorherigen Rahmens überlappt,
   iii. Verfolgen überlappender Objekte und zeitlicher Bewegungsvariationen, wobei das Verfolgen der überlappenden Objekte umfasst:

      a. Aktualisieren einer Geometrie von Objekten, die zwischen dem aktuellen und den vorherigen Rahmen überlappen, mit einer neuen Minimum-Bounding-Form und Verfolgen einer Änderung der Geschwindigkeit der Minimum-Bounding-Form,
      b. Hinzufügen einer beliebigen Gruppe, die nicht mit einer der Minimum-Bounding-Formen des vorherigen Rahmens überlappt, als neues Objekt und
      c. Entfernen jeglicher Objekte aus dem vorherigen Rahmen, die nicht mit einer Gruppe in dem aktuellen Rahmen überlappen.

2. Verfahren nach Anspruch 1, wobei die Bewegung von Subjekten eine Mikrokörperbewegung von Subjekten aufgrund von Herzschlag oder Atmung ist und ferner umfassend:
   Aggregieren (5) der Datenpunkte (10a, b, c; 11a, b, c) der Gruppe, um ein einzelnes Geschwindigkeitssignal (9) auszubilden, und Extrahieren einer Herzfrequenz oder einer Atemfrequenz aus dem einzelnen Geschwindigkeitssignal (9) der Gruppe (10a, b, c; 11a, b, c), wobei eine Fokuseinstellung des Radars (23) durch dynamisches Fokussieren auf einen Schwerpunkt der mindestens einen Gruppe (10a, b, c; 11a, b, c) vorgenommen wird.

3. Verfahren nach Anspruch 2, wobei die Aggregation von Datenpunkten eine Summierung von Punkten umfasst, die sich auf die Bewegungen großer Objekte beziehen.

4. Verfahren nach Anspruch 2 oder Anspruch 3, wobei die Aggregation (5) ein einzelnes zusammenfassendes Geschwindigkeitssignal umfasst, basierend auf der Mittelwertbildung einer Geschwindigkeit jedes Punkts der Gruppe (10a, b, c; 11a, b, c), dem Nehmen der mittleren Geschwindigkeit der Gruppe (10a, b, c; 11a, b, c), der Bestimmung der Maximalgeschwindigkeit der Gruppe, der Bestimmung der Minimalgeschwindigkeit der Gruppe (10a, b, c; 11a, b, c) oder der Bestimmung des Geschwindigkeitsbereich der Gruppe (10a, b, c; 11a, b, c) und Verwenden des Bereichs als das einzelne zusammenfassende Geschwindigkeitssignal (9).

5. Verfahren nach einem der Ansprüche 2 bis 4, wobei das Phased-Array-Radar (23) bei einer Frequenz von 60 - 81 GHz sendet, vorzugsweise zwischen 60 und 64 GHz und am meisten bevorzugt bei etwa 60 GHz.

6. Verfahren nach einem der Ansprüche 2 bis 5, wobei die 3D-Punktwolke einer Rauschfilterung (3) unterzogen wird, bevor sie in den Identifikationsalgorithmus (4) eingespeist wird.

**Revendications**

1. Procédé d'optimisation d'un ensemble de données provenant d'un radar à réseau phasé pour détecter le déplacement de sujets (20) dans une zone de couverture du radar, dans lequel ledit radar crée un ensemble de données de nuages de points 3D (2), ledit ensemble de données (2) étant introduit dans un algorithme d'identification d'objets (4) pour créer au moins un groupe de points de données (10a, b, c.) ; 11a, b, c) **caractérisé par** leur proximité et leur modèle de déplacement, définissant que ledit au moins un groupe (10a, b, c ; 11a, b, c) appartient à un seul sujet (20) et suivant le déplacement des points de données dudit groupe (10a, b, c.) ; 11a, b, c) ; dans lequel, après ledit regroupement de points, une forme de délimitation minimale, telle qu'une boîte, est définie autour dudit au moins un groupe (10a, b, c.) ; 11a, b, c) et le suivi de ladite forme limite minimale à travers des images de nuages de points 3D prises à différents moments consécutifs, dans lequel ledit suivi comprend les étapes suivantes :

   i. le calcul de la forme limite minimale pour chaque groupe de points dans un cadre,
   ii. la détermination si l'une quelconque des formes limites minimales trouvées dans une image courante chevauche l'une quelconque des formes limites minimales d'une image précédente,
   iii. le suivi des objets qui se chevauchent et des variations temporelles de déplacement, dans lequel le suivi des objets qui se chevauchent comprend :

      a. la mise à jour d'une géométrie d'objets qui se chevauchent entre les images actuelles et les images précédentes avec une nouvelle forme limite minimale et le suivi du changement de vitesse de cette forme limite minimale,
      b. l'ajout d'un groupe qui ne chevauche pas les formes minimales de la trame précédente comme nouvel objet, et
      c. le retrait de tout objet de ladite image précédente qui ne chevauche aucun groupe de ladite image actuelle.

2. Procédé selon la revendication 1, dans lequel le déplacement des sujets est un micro-déplacement du corps des sujets dû aux battements cardiaques ou à la respiration, et comprenant en outre :
   l'agrégation (5) des points de données (10a, b, c.) ; 11a, b, c) dudit groupe pour former un signal de vitesse unique (9) et l'extraction d'une fréquence cardiaque ou d'une fréquence respiratoire à partir du signal de vitesse unique (9) dudit groupe (10a, b, c ; 11a, b, c), dans lequel un réglage de mise au point dudit radar (23) est réaliser par une mise au point dynamique sur un centroïde dudit au moins un groupe (10a, b, c ; 11a, b, c).

3. Procédé selon la revendication 2, dans lequel l'agrégation des points de données comprend une addition de points liés à des déplacements d'objets importants.

4. Procédé selon la revendication 2 ou la revendication 3, dans lequel ladite agrégation (5) comprend un seul signal de vitesse sommaire sur la base de la moyenne d'une vitesse de chaque point dudit groupe (10a, b, c ; 11a, b, c), en prenant la vitesse médiane dudit groupe (10a, b, c ; 11a, b, c), en déterminant la vitesse maximale dudit groupe, en déterminant la vitesse minimale dudit groupe (10a, b, c ; 11a, b, c) ou en déterminant la plage de vitesses dudit groupe (10a, b, c ; 11a, b, c) et en utilisant la plage comme ledit signal unique de vitesse de synthèse (9).

5. Procédé selon l'une quelconque des revendications 2 à 4, dans lequel le radar à réseau phasé (23) émet à une fréquence comprise entre 60 et 81 GHz, de préférence entre 60 et 64 GHz et le plus préférablement autour de 60 GHz.

6. Procédé selon l'une quelconque des revendications 2 à 5, dans lequel ledit nuage de points 3D est soumis à un filtrage du bruit (3) avant d'être introduit dans ledit algorithme d'identification (4).

FIG.1

EP 4 244 647 B1

FIG.2

FIG.3

19

FIG.3a

FIG.4

FIG.5

FIG.6

◉ Desired focal point

FIG.7

FIG.8

FIG.9

FIG.10

FIG.11

FIG.12

FIG.13

FIG.14

FIG.15

FIG.16

FIG.17

2.3m

## FIG.18

1.0m

## FIG.19

| Experiment setup no. | Distance to chest (m) | Median difference (rpm) | IQR 75 difference (rpm) | IQR 95 difference (rpm) |
|---|---|---|---|---|
| 1 | 2.3 | 2 | 3 | 5 |
| 2 | 3 | 3 | 4 | 6 |
| 3 | 4 | 1 | 2 | 4 |
| 4 | 5 | 3 | 4 | 5 |
| 5 | 5.5 | 1 | 2 | 3 |
| 6 | 1.8 | 2 | 3 | 4 |
| 7 | 2.3 | 3 | 5 | 8 |
| 8 | 2.3 | 1 | 2 | 3 |

## FIG.20

FIG.21

FIG.22

FIG.23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102091974 B1
- US 8870785 B2
- KR 101895324 B1
- CN 209863802 U
- KR 102061589 B1
- US 2008294019 A
- US 2018078216 A
- US 2019209022 A
- GB 2449081 A

- WO 2017002103 A
- US 20150369911 A
- EP 3641649 A
- US 20200300972 A1
- CN 111142102
- US 2020237252 A
- US 2019094350 A
- US 2018279884 A

**Non-patent literature cited in the description**

- **Y. -C. TSAI et al.** High Accuracy Respiration and Heart Rate Detection Based on Artificial Neural Network Regression. *2020 42nd Annual International Conference of the IEEE Engineering in Medicine & Biology Society (EMBC),* 232-235
- **J. PEGORARO et al.** Multiperson Continuous Tracking and Identification From mm-Wave Micro-Doppler Signatures. *IEEE Transactions on Geoscience and Remote Sensing,* vol. 59 (4), ISSN 0196-2892, 2994-3009
- **P. ZHAO et al.** mID: Tracking and Identifying People with Millimeter Wave Radar. *2019 15th International Conference on Distributed Computing in Sensor Systems (DCOSS),* 33-40
- **SCHUBERT ; ERICH et al.** DBSCAN revisited, revisited: why and how you should (still) use DBSCAN. *ACM Transactions on Database Systems (TODS),* 2017, vol. 42 (3), 1-21
- **ANKERST ; MIHAEL ; MARKUS M. BREUNIG ; HANS-PETER KRIEGEL ; JÖRG SANDER.** OPTICS: Ordering points to identify the clustering structure. *ACM SIGMOID Record,* 1999, vol. 28 (2), 49-60
- **MCINNES et al.** hdbscan: Hierarchical density-based clustering. *Journal of Open Source Software,* vol. 2 (11), 205
- **DORIN COMANICIU ; PETER MEER.** Mean Shift: A robust approach toward feature space analysis. *IEEE Transactions on Pattern Analysis and Machine Intelligence.,* 2002, 603-619
- **BRENDAN J. FREY ; DELBERT DUECK.** Clustering by Passing Messages Between Data Points. *Science,* February 2007

- **BABADI ; BEHTASH ; EMERY N. BROWN.** A review of multitaper spectral analysis. *IEEE Transactions on Biomedical Engineering,* 2014, vol. 61 (5), 1555-1564
- **J. FRIEDMAN.** Greedy function approximation: a gradient boosting machine. *Annals of Statistics,* 2001, vol. 29 (5), 1189-1232
- LightGBM: a highly efficient gradient boosting decision tree. **GUOLIN KE ; QI MENG ; HOMAS FINLEY ; TAIFENG WANG ; WEI CHEN ; WEIDONG MA ; QIWEI YE ; TIE-YAN LIU.** Proceedings of the 31st International Conference on Neural Information Processing Systems (NIPS'17). Curran Associates Inc, 2017, 3149-3157
- **TIANQI CHEN ; CARLOS GUESTRIN.** XGBoost: A Scalable Decision Tree Boosting System. *22nd SIGKDD Conference on Knowledge Discovery and Data Mining,* 2016
- **YOAV FREUND ; ROBERT E. SCHAPIRE.** A decision-theoretic generalization of on-line learning and an application to boosting. *Journal of Computer and System Sciences,* August 1997, vol. 55 (1), 119-139
- **ORENKOVA ; LIUDMILA et al.** CatBoost: Unbiased boosting with categorical features. *Advances in neural information processing systems,* 2018
- **OPITZ ; DAVID ; RICHARD MACLIN.** Popular ensemble methods: An empirical study. *Journal of artificial intelligence research,* 1999, vol. 11, 169-198